# EUROPEAN PATENT SPECIFICATION

(11) **EP 3 474 978 B1**
(45) Date of publication and mention of the grant of the patent: **13.10.2021**
(21) Application number: 17816316.8
(22) Date of filing: 23.06.2017
(51) Int. Cl.: B01J 8/18, B01J 19/12, C01B 13/34, C01B 13/14, C04B 35/626, H01M 4/52, H01M 4/50, H01M 4/48, C01B 25/45, C01G 23/00, C01G 25/00, C01G 45/12, C01G 49/00, C01G 53/00, H01J 37/32, H01M 4/505, H01M 4/525

(54) **LITHIUM ION BATTERY MATERIALS**
LITHIUM-IONEN-BATTERIE-MATERIALIEN
MATÉRIAUX DE BATTERIE AU LITHIUM-ION

(30) Priority: 23.06.2016 US 201662353663 P
(43) Date of publication of application: 01.05.2019
(73) Proprietor: 6K Inc., North Andover, MA 01845 (US)
(72) Inventor: HADIDI, Kamal, Somerville, MA 02143 (US); WROBEL, Gregory, Groveland, MA 01834 (US)
(74) Representative: Forresters IP LLP
(86) International application number: PCT/US2017/039049
(87) International publication number: WO 2017/223482

(56) References cited:
- WO-A1-2016/048862
- US-A1- 2004 022 939
- US-A1- 2010 086 853
- US-A1- 2012 077 082
- US-A1- 2014 217 630
- US-A1- 2014 328 724
- US-B1- 6 838 072
- SHIH-MIN CHANG ET AL: "One-Step Fast Synthesis of Li4Ti5O12 Particles Using an Atmospheric Pressure Plasma Jet", JOURNAL OF THE AMERICAN CERAMIC SOCIETY., vol. 97, no. 3, 26 December 2013 (2013-12-26), pages 708-712, XP055659201, US ISSN: 0002-7820, DOI: 10.1111/jace.12728
- BABAJIDE PATRICK AJAYI ET AL: "A rapid and scalable method for making mixed metal oxide alloys for enabling accelerated materials discovery", JOURNAL OF MATERIALS RESEARCH, vol. 31, no. 11, 28 March 2016 (2016-03-28), pages 1596-1607, XP055658437, US ISSN: 0884-2914, DOI: 10.1557/jmr.2016.92
- EDWARD K. NYUTU ET AL: "Ultrasonic Nozzle Spray in Situ Mixing and Microwave-Assisted Preparation of Nanocrystalline Spinel Metal Oxides: Nickel Ferrite and Zinc Aluminate", JOURNAL OF PHYSICAL CHEMISTRY C, vol. 112, no. 5, 1 February 2008 (2008-02-01), pages 1407-1414, XP055658455, US ISSN: 1932-7447, DOI: 10.1021/jp075647l

## Description

### TECHNICAL FIELD

The present disclosure relates to techniques for preparing battery materials, and more specifically to techniques for generating lithium ion (Li-ion) battery materials.

### BACKGROUND OF THE TECHNOLOGY

As portable electronic devices steadily decrease in size, the need for smaller and lighter batteries that can provide power to these devices increases. Demand for higher energy batteries is also increasing in the field of hybrid and fully electric vehicles. Such vehicles can improve air quality by reducing air pollution and vehicular emissions caused by traditional combustion engines. Rechargeable Li-ion batteries can be used in both consumer electronics and electric vehicle applications. However, expensive and complicated manufacturing processes continue to contribute to the high cost of lithium-containing materials commonly used in Li-ion batteries and the Li-ion batteries.

Li-ion batteries generally contain a negative electrode, known as an anode, a positive electrode, known as a cathode, and an electrolyte between the anode and the cathode. During charging, Li-ions (Li+) migrate from the cathode through the electrolyte and intercalate within the structure of the anode. Graphite can be used as an intercalation compound for the anode because the Li-ions can be embedded within the van der Waals gap between layers of the graphite, where they are stored until discharge. An organic solution including a lithium salt or Li-ion conducting polymer can be used as the electrolyte, in some cases.

In Li-ion batteries, single-phase materials containing appropriate amounts of lithium transition metal oxide are desirable for the cathode, or positive electrode. Examples of such single-phase materials include LiNi_{0.8}Co_{0.2}O₂, LiCoO₂, LiNiₓMn_{y}Co_{z}O₂ or LiNiₓCo_{y}Al_{z}O₂. Specifically with respect to LiNiₓMn_{y}Co_{z}O₂, varying the content ratio of manganese, nickel, and cobalt can tune the power and energy performance of a battery. However, the production of these single-phase transition metal oxides can require time and energy consuming processing steps, such as ball milling, wet milling, sintering, washing, mixing, grinding, etc. In some cases, alkaline solutions are used in one or more initial processing steps, which can produce unwanted by products. The complexity of such processes contribute to the high cost of Li-ion batteries. In addition, tailoring or optimizing the stoichiometry of the Li-ion battery materials may be difficult or impossible to accomplish on a large or commercial scale. Chang *et al* discloses a one-step process to fabricate crystalline Li₄Ti₅O₁₂ particles. Ajayi *et al* discloses a method for making mixed metal oxide alloys. US 6,838,072 B1 discloses a process for preparing lithium intercalation compounds. Nyutu *et al* discloses a method for the preparation of nanocrystalline spinel metal oxides.

### SUMMARY

The invention is defined in the claims. Exemplary embodiments of the present technology are directed to systems and methods for generating lithium-containing particles and for tailoring Li-ion battery materials. In one aspect, the present technology relates to a method for generating lithium ion battery materials. The method includes combining starting materials to form a homogeneous precursor solution including lithium and generating droplets with controlled size of the homogeneous precursor solution using a droplet maker. The method also includes introducing the droplets of the homogeneous precursor solution into a microwave generated plasma, producing micron or sub-micron scale lithium-containing particles from the microwave generated plasma, collecting the lithium-containing particles, and forming a slurry with the lithium-containing particles to form lithium ion battery materials.

Embodiments of this aspect of the technology can include one or more of the following features. In some embodiments, collecting the lithium-containing particles includes quenching the lithium-containing particles, and the method also includes controlling a quenching rate of the lithium-containing particles by selecting a quenching fluid, controlling a quenching fluid flow velocity, or controlling a quenching fluid temperature. In some embodiments, the method also includes controlling a size of the droplets of the homogeneous precursor solution using the droplet maker. In some embodiments, the method also includes controlling a residence time of the droplets within the microwave generated plasma by controlling a plasma gas flow velocity, a power density of the microwave generated plasma, and/or a velocity of the droplets exiting the droplet maker. In some embodiments, the homogeneous precursor solution includes an aqueous solution of hydrated or non-hydrated forms of lithium acetate, nickel acetate, manganese acetate, and cobalt acetate. In other embodiments, the homogeneous precursor solution includes an aqueous solution of lithium nitrate, nickel nitrate, manganese nitrate, and cobalt nitrate. In some embodiments, generating droplets with controlled size includes generating two or more streams of droplets having different diameters. In some embodiments, the microwave generated plasma is generated in oxygen gas or an oxygen-containing gas.

In another aspect, the present technology relates to a method of tailoring lithium ion battery materials. The method includes combining starting materials to form a homogeneous precursor solution including lithium and generating droplets with controlled size of the homogeneous precursor solution using a droplet maker. The method also includes introducing the droplets of the homogeneous precursor solution into a microwave generated plasma, producing micron or sub-micron scale lithium-containing particles from the microwave generated plasma, and quenching the lithium-containing particles. The method also includes tailoring the porosity, morphology, particle size, particle size distribution, or chemical composition of the lithium-containing particles by controlling precursor solution chemistry, droplet size, plasma gas flow rates, residence time of the droplets within the microwave generated plasma, quenching rate, or power density of the microwave generated plasma.

Embodiments of this aspect of the technology can include one or more of the following features. In some embodiments, tailoring the morphology of the lithium-containing particles includes controlling the residence time of the droplets within the microwave generated plasma and an afterglow region of the microwave generated plasma. In some embodiments, controlling the porosity of the lithium ion particles includes controlling amounts of nitrate materials and acetate materials within the homogeneous precursor solution, controlling the solution precursor chemistry, or controlling the residence time of the droplets within the microwave generated plasma. In some embodiments, controlling the chemical composition of the lithium-containing particles includes controlling proportions of the starting materials within the precursor solution. In some embodiments, controlling the particle size of the lithium ion particles includes controlling the droplet size of the droplets of the precursor solution, or controlling a concentration of starting materials within the precursor solution. In some embodiments, generating droplets with controlled size includes generating two or more streams of droplets having different diameters. In one such embodiment, the two or more streams of droplets are generated using different nozzles or openings in the droplet maker. In some embodiments, tailoring the chemical composition of the lithium ion particles includes determining a desired chemical composition of the lithium ion particles prior to forming the homogeneous precursor solution, and calculating stoichiometric proportions of the starting materials based on the desired chemical composition of the lithium ion particles.

In another aspect, the present technology relates to a method of preparing lithium-containing particles represented by the formula: LiNiₓMn_{y}Co_{z}O₂, wherein x ≥ 0, y ≥ 0, z ≥ 0, and x+y+z=1. The method includes dissolving a combination of lithium salt, nickel salt, manganese salt, and cobalt salt in a solvent to form a homogeneous precursor solution, generating droplets with controlled size of the homogeneous precursor solution using a droplet maker, introducing the droplets into a microwave generated plasma, producing micron or sub-micron scale particles of LiNiₓMn_{y}Co_{z}O₂ from the microwave generated plasma, and collecting the particles of LiNiₓMn_{y}Co_{z}O₂. In one embodiment of this aspect of the technology, generating droplets with controlled size includes generating two or more streams of droplets having different diameters in order to generate a multi-modal particle size distribution among the particles of LiNiₓMn_{y}Co_{z}O₂.

In another aspect, the present technology relates to a method for preparing lithium-containing particles represented by the formula: LiNiₓCo_{y}Al_{z}O₂, wherein x = ~ 0.8, y = -0.15, z = ~ 0.05. The method includes dissolving a combination of lithium salt, nickel salt, cobalt salt, and aluminum salt in a solvent to form a homogeneous precursor solution, generating uniformly sized droplets of the homogeneous precursor solution using a droplet maker, introducing the uniformly sized droplets into a microwave generated plasma, producing micron or sub-micron scale particles of LiNiₓCo_{y}Al_{z}O₂ from the microwave generated plasma, and collecting the particles of LiNiₓCo_{y}Al_{z}O₂. In one embodiment of this aspect of the technology, generating droplets with controlled size includes generating two or more streams of droplets having different diameters in order to generate a multi-modal particle size distribution among the particles of LiNiₓCo_{y}Al_{z}O₂.

The above aspects of the present technology provide one or more of the following advantages. Some embodiments allow for the production of lithium-containing particles in a matter of seconds or minutes, rather than hours or days. Some embodiments allow for the production of lithium-containing particles without the production or with limited production of harmful or toxic byproducts, or without the loss of a significant proportion of the starting materials. Some embodiments also allow for the production of lithium-containing particles of different chemical compositions and different characteristics (e.g., different densities or morphologies) using a single production systems. Some embodiments also allow for the precise tailoring of particular lithium-containing particles by controlling or adjusting various process parameters. Some embodiments also allow for the production of lithium-containing particles with controlled size distribution. Some embodiments also allow for the production of lithium-containing particles with a controlled single particle size distribution, a bimodal size distribution, or a multimodal particle size distribution. Some embodiments allow for the production of lithium-containing particles with a surface coating that can be of a different material such as carbon, alumina, etc.

### BRIEF DESCRIPTION OF THE DRAWINGS

The foregoing and other features and advantages provided by the present disclosure will be more fully understood from the following description of exemplary embodiments when read together with the accompanying drawings, in which:
FIG. 1 is a flow chart of an exemplary method for generating lithium-containing particles, according to an embodiment of the present disclosure.
FIG. 2 shows a comparison between a conventional batch processing technique 10 for producing lithium-containing particles, and the continuous flow process 20 described in the present disclosure
FIG. 3 is a flow chart of an exemplary method for tailoring Li--ion battery materials, according to an embodiment of the present disclosure.
FIG. 4 illustrates an example system for generating lithium-containing particles using a microwave generated plasma, according to an exemplary embodiment.
FIG. 5 is a block diagram illustrating a system for tailoring Li-ion battery materials, according to an exemplary embodiment.
FIG. 6 is a graph of the X-ray diffraction pattern of lithium-containing particles prepared according to an embodiment of the present disclosure.
FIG. 7 shows an enlarged image of the example lithium-containing particles of FIG. 6.

### DETAILED DESCRIPTION OF THE TECHNOLOGY

The invention is defined in the claims. Provided herein are methodologies, systems, and apparatus for producing lithium-containing particles and Li-ion battery materials. Cathode materials for Li-ion batteries can include lithium-containing transition metal oxides, such as, for example, LiNiₓMn_{y}Co_{z}O₂ or LiNiₓCo_{y}Al_{z}O₂. These materials contain a layered crystal structure where layers of lithium atoms sit between layers of transition-metal oxide polyhedra. As Li-ions deintercalate from the crystal structure, charge neutrality is maintained with an increase in the valence state of the transition metals. LiNiₓMn_{y}Co_{z}O₂ or LiNiₓCo_{y}Al_{z}O₂ possess desirable characteristics such as relatively high energy density (mAh/g), high cyclability (% degradation per charge/discharge cycle), and thermal stability (≤100°C).

According to conventional techniques, generating lithium-containing particles for use in Li-ion batteries can require between 8-10 process steps lasting from hours to days. According to some techniques, the starting materials for the lithium-containing particles must be stirred, precipitated, filtered, washed, dried, sieved, mixed, calcinated, classified, and coated all before a slurry can be formed.

Solid-state processes for producing lithium-containing particles are generally multi-step processes requiring the crushing of stoichiometric proportions of solid precursors, followed by high temperature diffusion reaction to form the final structure. Such processes often produce large and irregularly shaped particles that exhibit phase inhomogeneity, non-uniform size distribution, and low surface area leading to increased resistance of the Li-ion diffusion pathway. Post-processing reduction of particle size is often required to increase the surface area and minimize the Li-ion diffusion pathway length. To overcome the sliding friction and agglomeration between irregularly shaped particles when tape casting the cathode material onto the current collector requires the use of expensive and toxic solvents, such as N-methyl pyrrolidone. Such solvents increase the complexity and cost of the manufacturing process. Further, multiple and long processing steps increases the risk of contaminants which results in decreased purity.

Synthesis of, for example, LiNi_{1/3}Mn_{1/3}Co_{1/3}O₂ through co-precipitation is conventionally a multi-step process involving solution reaction of NiSO₄, CoSO₄, and MnS04. The resulting (Ni_{1/3}Mn_{1/3}Co_{1/3})(OH)₂ are dried at approximately 105°C, followed by reaction with LiOH^{∗}H₂O) at approximately 500°C, and then calcination at between 900-1000°C for up to about 10 hours. The resulting powders have a broad size distribution including spherical, semi-spherical, and irregularly shaped particles. The chemical waste solution contains sulfites and strong bases which require special handling and disposal which increases cost.

Spray pyrolysis techniques may also be used to synthesize Li-ion cathode materials. An example spray pyrolysis method can be used to synthesize LiNi_{1/3}Mn_{1/3}Co_{1/3}O₂ starting with an aqueous precursor solution of LiNO₃, Ni(NO₃), Co(NO₃)₂, and Mn(NO₃)₂. The precursor solution is atomized using an ultrasonic atomizer and exposed to ~500°C using a furnace or flame where the precursor solution is evaporated and decomposed into the desired LiNi_{1/3}Mn_{1/3}Co_{1/3}O₂ particles. However, such techniques can produce toxic or unwanted byproducts. For example, the use of nitrates during spray pyrolysis produces NOₓ. In addition, high-pressure and ultrasonic atomizers can produce considerable size distributions leading to a size-dependent thermal history of the resulting particles which may lead to phase and morphology inhomogeneities.

According to the techniques described in this disclosure, lithium-containing particles can be produced using a single flow process that can be completed in hundreds of milliseconds, rather than hours or days. Not only is the process for generating lithium-containing particles significantly simplified and accelerated, the end products are significantly more uniform in size, and the porosity, morphology, and chemical composition can be precisely tailored. The techniques described herein may be used to produce cathode, anode, or solid electrolyte materials for lithium based batteries. Exemplary materials for use in one or more of the cathode, anode, and electrolyte include, but are by no means limited to: LiNiₓMn_{y}Co_{z}O₂, LiNiₓCo_{y}Al_{z}O₂, LiFePO₄, Li₈ZrO₆, Li₂FeMn₃O₈, Li₄Ti₅, O₁₂, SnO₂, Co₉S₈, LiVP₂O₇, NaLaTi₂O₆, LiₓPO_{y}N_{z}, Li garnet, and Li₁₀GeP₂O₁₂.

The combination of chemically homogeneous and uniformly size-controlled droplet feedstock solution and homogeneous thermal processing provides distinct advantages over conventional solid-state, co-precipitation, and spray pyrolysis processing techniques. In one embodiment of the present disclosure, a homogeneous precursor solution is mixed at the molecular level to ensure equal distribution of starting materials within the solution. The precursor solution is formed into droplets using a droplet maker that can generate one or more streams of droplets having precisely controlled sizes. In some embodiments, the droplet maker can be a piezoelectric droplet maker, such as the droplet maker described in U.S. Patent No. 9,321,071 and U.S. Patent Publication No. 2016/0228903. In one particular embodiment, the droplet maker can control the size of the droplets to a precise diameter with a size distribution of about ±2%. In some embodiments, the droplet maker can include nozzles or openings having different sizes in order to generate streams of droplets having different diameters, which may produce a multi-modal particle size distribution in the end particles. The droplets of precursor solution can then be axially injected into a plasma as a single stream or several linear streams of droplets. The plasma can include, for example, an axisymmetric microwave generated plasma with laminar gas flow and a substantially uniform temperature profile. Examples of such a microwave generated plasma can be found in U.S. Patent No. 8,748,785, U.S. Patent No. 8,951.496, U.S. Patent No. 9,023,259, and U.S. Patent Publication No. 2013/0270261. Each of the droplets follow an identical thermal path through the laminar plasma, ensuring an identical thermal history for each particle produced and resulting in a substantially consistent final composition.

In another example embodiment, the techniques described herein can be used to produce lithium-containing materials, such as LiNiₓMn_{y}Co₂O₂ (where x≥0, y≥0, z≥0, and x+y+z=1) and LiNiₓCo_{y}Al_{z}O₂ (where x = ~0.8, y = ~0.15, z = ~0.05) positive cathode powders in a single processing step without the need for post-processing. Various characteristics of the final lithium-containing particles, such as porosity, particle size, particle size distribution, phase composition and purity, microstructure, etc. can be tailored and precisely controlled by fine tuning various process parameters. In some embodiments, these process parameters can include precursor solution chemistry, droplet size, plasma gas flow rates, plasma process atmosphere, residence time of the droplets within the plasma, quenching rate, power density of the plasma, etc. These process parameters can be tailored, in some embodiments, to produce micron and/or sub-micron scale particles with high surface area, a specific porosity level, low-resistance Li-ion diffusion pathway, a narrow size distribution of about ±2%, and containing a nano-grain microstructure. The residence time of the droplets within the plasma can be controlled, in some embodiments, by controlling the plasma gas flow rate and/or controlling the power density of the microwave generated plasma. In some embodiments, the quenching rate can be adjusted by selecting a different quenching fluid, such as nitrogen, oxygen, or helium. For example, helium can provide a higher quenching rate than other fluids, but may add significant costs to the production process. Different characteristics of the plasma can be adjusted, in some cases, by controlling the plasma process atmosphere, which can include 0 2 or various mixtures of oxygen, argon, helium, etc.

**In** some embodiments, the use of nitrates in the precursor solution can result in more porous lithium-containing particles because nitrates can react with the heat of the microwave generated plasma to cause an exothermic reaction and rapid release of gasses, which results in porosity. In other embodiments, the use of acetates in the precursor solution can result in more dense lithium-containing particles, compared to when nitrates are used, because acetates do not react with the heat of the microwave generated plasma in the same way as nitrates. Furthermore, the use of acetates in the precursor solution chemistry generates no NOₓ emissions or hazardous waste and can be run continuously. As will be appreciated, various mixtures and proportions of nitrates and acetates can be used in the precursor solution in order to tailor the lithium-containing particles to a desired porosity.

**In** one example embodiment, LiNi_{1/3}Mn_{1/3}Co_{1/3}O₂ (NMC-333) can be produced using a precursor solution that includes an aqueous solution of 1 mol lithium acetate [Li(COOCH₃)], 0.33 mol of nickel acetate tetrahydrate [Ni(COOCH₃)₂^{∗}4H₂O], 0.33 mol manganese acetate tetrahydrate [Mn(COOCH₃)₂^{∗}4H₂O], and 0.33 mol cobalt acetate tetrahydrate [Co(COOCH₃)₂^{∗}4H₂O)]. This precursor solution is mixed into a homogeneous solution and formed into droplets with controlled size using a droplet maker, as described above. The droplets of the precursor solution can then be introduced axially to a microwave generated plasma, where the liquid is evaporated, the acetates decompose, and the remaining transition-metal cations react with the oxygen-containing plasma to yield spherical ceramic particles of the desired stoichiometry.

**In** alternative embodiments, different chemical compositions of LiNiₓMn_{y}CO_{z}O₂ can be produced by tailoring the proportions of starting materials in the homogeneous precursor solution. For example, LiNi_{0.8}Mn_{0.3}Co_{0.2}O₂ (NMC-532), LiNi_{0.6}Mn_{0.2}Co_{0.2}O₂ (NMC-622), or LiNi_{0.8}Mn_{0.1}Co_{0.1}O₂ (NMC-811) can be produced by providing different proportions of lithium, nickel, manganese, and cobalt salts to the precursor solution.

Other lithium-containing materials, such as LiPON or Li₁₀GeP₂O₁₂, can be produced using the techniques described herein. For example, a target stoichiometry of Li₃PO₄₋ₓNₓ can be produced by using a precursor solution including a homogeneous mixture of lithium nitrate and ammonium phosphate. Additional materials that may be produced using the techniques described in this disclosure include LMO (which can exist as LiMnO₂, Li₂Mn₂O₄, or Li_{1.12}Mn_{1.88}O₂), LTO (which exhibits the formula Li₄Ti₅O₁₂), LiVP₂O₇, LiFePO₄, Li₈ZrO₆, Li₂FeMn₃O₈, etc.

According to some embodiments, the techniques and systems described in this disclosure can be used to create core-shell structures, such as carbon coated particles (e.g., lithium-containing particles coated with carbon). Other coatings or shells, such as alumina coatings, can also be produced using the techniques described herein. In other embodiments, different types of battery materials can be coated or layered onto a substrate, such as the current collector of a battery. These materials can be deposited in discrete layers having desired thicknesses, or as a continuously graded coating where the material composition of the coating gradually changes throughout the thickness of the coating. These different materials can be deposited by controlling the composition of the initial precursor solution, in some embodiments.

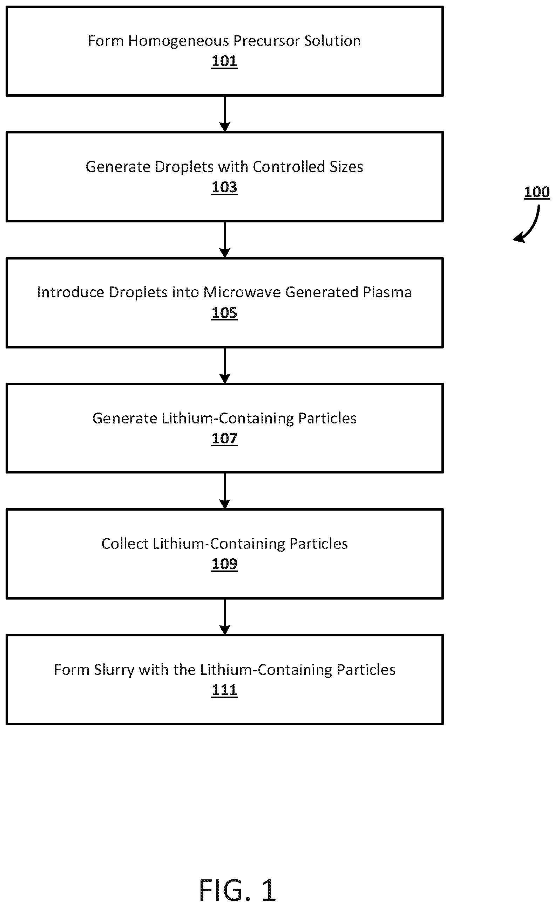

FIG. 1 is a flow chart 100 of an exemplary method for generating lithium-containing particles, according to an embodiment of the present disclosure. In step 101, a number of starting materials are combined to form a homogeneous precursor solution including lithium. In some embodiments, the homogeneous precursor solution is mixed at the molecular level such that there is an equal distribution of the starting materials within the precursor solution. For example, the homogeneous precursor solution can include an aqueous solution made of metallic salts such as lithium acetate, nickel acetate tetrahydrate, manganese acetate tetrahydrate, and cobalt acetate tetrahydrate all dissolved in a solvent such as water. In another example, the homogeneous precursor solution can include an aqueous solution made of metallic salts such as lithium nitrate, nickel nitrate, manganese nitrate, and cobalt nitrate, all dissolved in a solvent such as water. As discussed above, different stoichiometric proportions of the starting materials can result in different chemical compositions in the final lithium-containing particles.

**In** step 103, droplets of the homogeneous precursor solution are generated with a controlled size using a droplet maker. The droplet maker can be, for example, a piezoelectric droplet maker that is configured to generate one or more streams of droplets having precise sizes and diameters. In some embodiments, the size of the droplets is determined based on the size and/or geometry of capillary nozzles or holes in a capillary plate of the droplet maker. The droplet maker can be configured to generate one or more streams of droplets having the same size, or various streams of droplets having different sizes and diameters. In one particular embodiment, the droplet maker can control the size of the droplets to a precise diameter with a size distribution of about ±2%.

**In** step 105, the droplets of the homogeneous precursor solution are introduced into a microwave generated plasma. In some embodiments, the droplets can be injected axially into a microwave plasma torch such that each of the droplets within the droplet stream generated by the droplet maker is exposed to the same temperature profile. As discussed above, the plasma can include an axisymmetric microwave generated plasma with laminar gas flow, such that the droplets are exposed to a consistent thermal path within the plasma.

In step 107, micron and sub-micron scale lithium-containing particles are produced from the microwave generated plasma. In one example embodiment, as the droplets pass through the microwave generated plasma, the liquid is evaporated, the acetates and/or nitrates within the precursor solution decompose, and the remaining transition-metals react with the plasma and yield spherical ceramic particles of the desired chemical composition. As discussed above, various characteristics of the lithium-containing particles can be tailored by controlling the different process parameters such as residence time of the droplets within the microwave generated plasma and others. In some embodiments, the residence time can be controlled by adjusting the plasma gas flow velocity, the power density of the microwave generated plasma, and/or the velocity of the droplets exiting the droplet maker.

**In** step 109, the lithium-containing particles are collected. In some embodiments, collecting the lithium-containing particles includes quenching the particles using, for example, a quenching chamber. The quenching rate of the lithium-containing particles can be controlled, in some embodiments, by selecting a quenching fluid, controlling the flow speed of the quenching fluid, or controlling the quenching fluid temperature. Examples of quenching fluids include oxygen, nitrogen, liquid nitrogen, or helium. In another embodiment, the lithium-containing particles are naturally quenched into a chamber without the addition of any gas. In alternative embodiments, the lithium-containing particles described herein can be coated onto a substrate material directly from the plasma.

Once the lithium-containing particles have been collected, a slurry is formed in step 111 with the lithium-containing particles to form Li-ion battery materials.

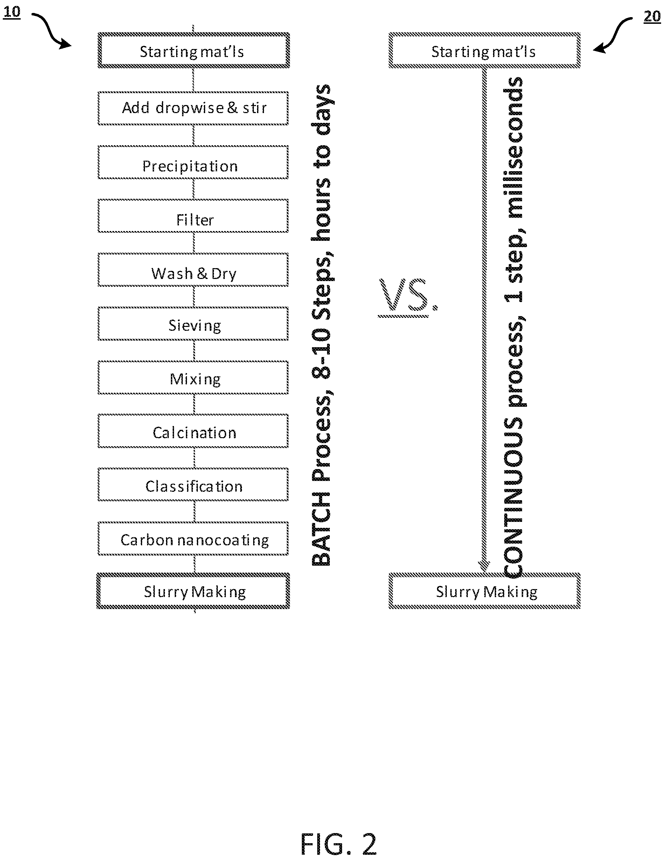

FIG. 2 shows a comparison between a conventional batch processing technique 10 for producing lithium-containing particles, and the continuous flow process 20 described in the present disclosure. According to a conventional batch processing technique 10, an amount of starting materials must undergo a number of discrete processing steps, requiring different machinery and chemical reactions. These steps can include, for example, stirring, precipitation, filtering, washing, drying, sieving, mixing, calcination, classification, and coated all before a slurry can be formed. Such techniques can take up to days to complete for each batch of starting materials. In contrast, the continuous process flow 20 described in the present disclosure can form lithium-containing particles from a set of starting materials in a single continuous step that can be completed on a scale of milliseconds to minutes. Specifically, once the starting materials have been dissolved into an aqueous precursor solution, droplets of the precursor solution are introduced into a plasma torch that generates the lithium-containing particles in a single step. The reactions that take place within the plasma torch, the various components of the system, as well as examples of how the precursor solution can be made, are described in more detail with respect to FIGS. 1, 4, and 5.

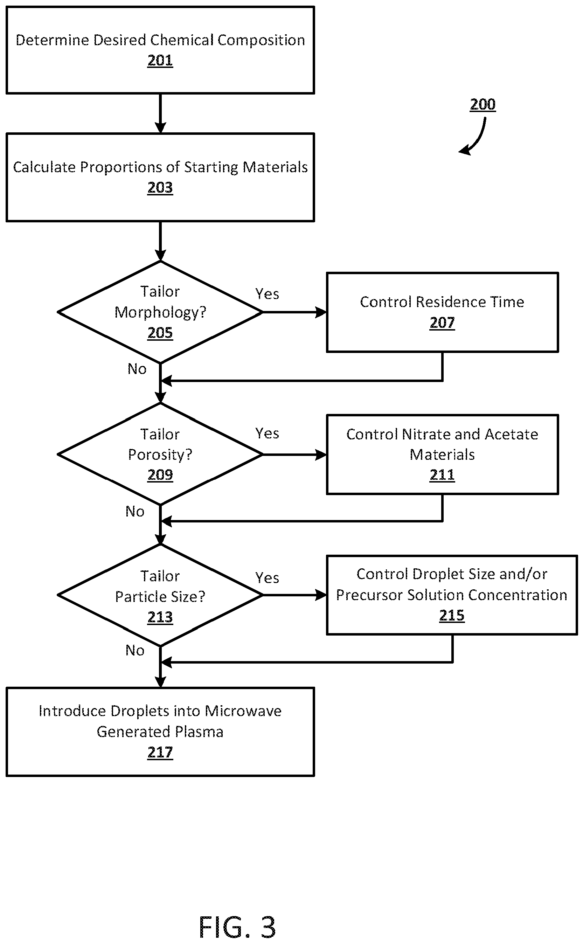

**In** addition to providing the advantages of much faster processing time (minutes vs. days), embodiments of the present technology allow for the customization or tailoring of various material properties. FIG. 3 is a flow chart 200 of an exemplary method for tailoring Li--ion battery materials, according to an embodiment of the present disclosure. Step 201 involves determining a desired chemical composition of the lithium-containing particles prior to forming the homogeneous precursor solution. In some embodiments, the techniques disclosed herein can be used to produce various lithium-containing particles having different chemical compositions, such as NMC-333, NMC-532, NMC-622, NMC-811. As discussed above, each of these lithium-containing particles can be produced using acetates and/or nitrates, or other chemicals in the precursor solution, depending on the desired properties of the end particles. In other embodiments, various chemical compositions of LiNiₓCo_{x,}AlₓO₂ or Li₃PO₄₋ₓNₓ can also be produced.

Once the desired chemical composition of the lithium-containing particles is determined, stoichiometric proportions of the starting materials are calculated in step 203. These proportions are based on the desired chemical composition of the end particles, and can be precisely tailored to produce the desired particles. In one example embodiment, in order to produce NMC-333, the starting materials can include 1 mol lithium acetate [Li(COOCH₃)], 0.33 mol nickel acetate tetrahydrate [Ni(COOCH₃)₂^{∗}4H₂O], 0.33 mol manganese acetate tetrahydrate [Mn(COOCH₃)₂^{∗}4H₂O], and 0.33 mol cobalt acetate tetrahydrate [CO(COOCH₃)₂^{∗}4H₂O)]. In another example, the starting materials for producing NMC-333 can include 1 mol lithium nitrate [LiNO_{3]}, 0.33 mol nickel nitrate [Ni(NO₃)₂], 0.33 mol manganese nitrate [Mn(NO₃)₂], and 0.33 mol cobalt nitrate [Co(NO₃)₂]. Different stoichiometric proportions of the starting materials can be calculated in order to produce NMC-532, NMC-622, NMC-811, etc.

Step 205 determines whether the morphology of the lithium-containing particles should be tailored. If the morphology is to be tailored, the method continues to step 207, where the residence time of the droplets within the microwave generated plasma is controlled in order to tailor the morphology of the lithium-containing particles. For example, an amorphous phase can be minimized or eliminated by increasing the residence time at a particular temperature, in some embodiments. The residence time can be tailored, in some embodiments, by controlling the flow velocity of the plasma gas, the power density of the microwave generated plasma, and/or the velocity of the precursor droplets exiting the droplet maker.

The method then continues to step 209 to determine whether the porosity of the lithium-containing particles should be tailored. If the porosity is to be tailored, the method continues in step 211 with controlling an amount of nitrate materials and acetate materials within the precursor solution, controlling the solution precursor chemistry, or controlling the residence time of the droplets within the microwave generated plasma. As discussed above, the use of nitrates in the precursor solution can result in more porous lithium-containing particles, while the use of acetates in the precursor solution can result in more dense lithium-containing particles. As will be appreciated, various mixtures and proportions of nitrates and acetates can be used in the precursor solution in order to tailor the lithium-containing particles to a desired porosity.

The method then continues to step 213 to determine whether the particle size of the lithium-containing particles should be tailored. If the particle size is to be tailored, the method continues in step 215 with controlling the droplet size of the droplets of the homogeneous precursor solution or controlling a concentration of the starting materials within the homogeneous precursor solution. For example, if the droplets include an increased concentration of starting materials, the resulting lithium-containing particles will be larger because a larger concentration of solid materials will be available to form the particles once the liquid within the solution evaporates. In some embodiments, various sized lithium-containing particles can be produced in the same process flow by generating different streams of droplets having different sizes.

The method then continues to step 217 with introducing the droplets into the microwave generated plasma at the desired parameters in order to produce the tailored lithium-containing particles. Once the tailored lithium-containing particles have been produced, they may be quenched and/or collected, as discussed above in reference to FIG. 1.

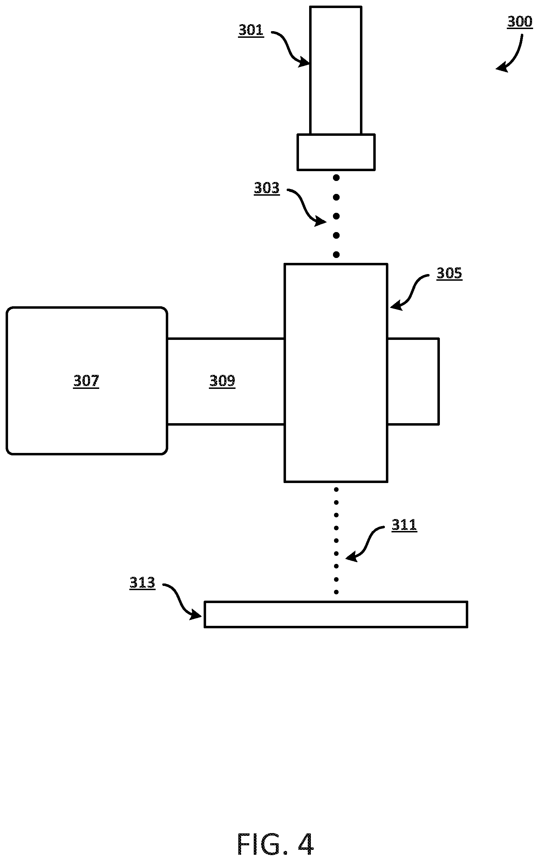

FIG. 4 illustrates a system 300 for generating lithium-containing particles 311 using a microwave generated plasma, according to an exemplary embodiment. In this example embodiment, a piezoelectric droplet maker 301 generates a stream of droplets 303 of a lithium-containing precursor solution having controlled droplet sizes. Each droplet 303 has a precisely controlled size and concentration of starting materials in order to produce the desired end particles, as discussed above. A microwave radiation source 307 provides microwave radiation via a waveguide 309 in order to generate a plasma within a plasma chamber 305. The stream of droplets 303 is introduced axially to the plasma chamber 305 and each droplet is exposed to a substantially uniform temperature profile within the plasma chamber 305. As discussed above, the microwave generated plasma produces lithium-containing particles 311, which exit the plasma chamber 305 and are collected using a particle collector 313.

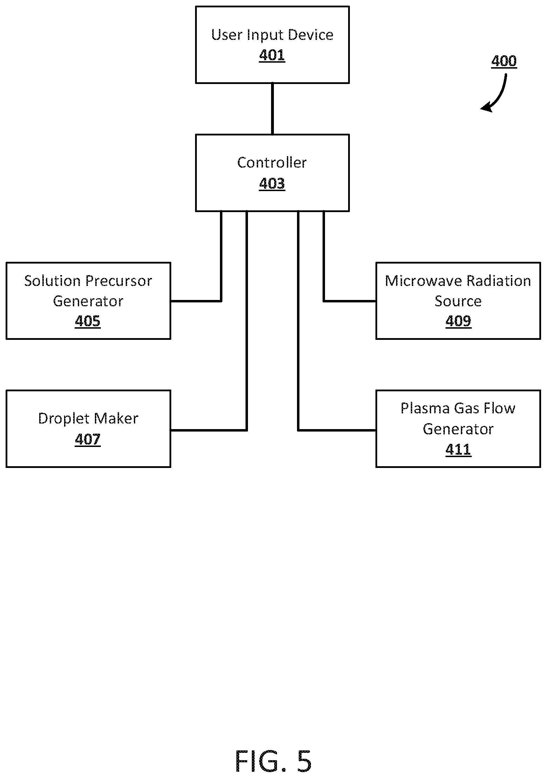

**FIG.** 5 is a block diagram 400 illustrating a system for tailoring Li-ion battery materials, according to an exemplary embodiment. In this particular embodiment, a user input device 401 can receive input from a user indicating the desired parameters of the lithium-containing particles to be produced. For example, the user input device 401 can receive instructions to produce NMC-532 particles having a desired porosity, morphology, and particle size. The controller 403 can receive the desired particle parameters from the user input device and control (e.g., by accessing a database or look-up table, or executing control processes associated with different inputs) various components and precursors of the system. For example, the controller 403 can receive a desired set of characteristics and can select an appropriate set of starting materials for the precursor solution in order to produce the desired particles. In some cases, the controller 403 can determine a type of starting material based on price, the type of plasma gas that will be used, the desired porosity of the end particles, etc. For example, the controller may select which types of starting materials will be used in the precursor solution in order to limit overall production costs, increase particle density, or limit toxic byproducts. In some embodiments, the controller 403 can also calculate a desired proportion and concentration for the precursor solution and control the solution precursor generator 405 to generate the desired solution. The controller 403 can also calculate a desired droplet size and control the droplet maker 407 to generate droplets of the desired size. In some cases, the controller 403 can control the droplet maker 407 in order to adjust the speed of the droplets exiting the droplet maker 407. In some embodiments, the controller 403 can control the plasma gas flow generator 411 in order to adjust the plasma gas flow velocity. In other embodiments, the controller 403 can control the microwave radiation source 409 in order to adjust the power density or the temperature of the microwave generated plasma. For example, the microwave radiation source 409 can be controlled to produce a substantially uniform temperature profile of about 6,000 K. The quantities and proportions of starting materials can be selected, in some embodiments, according to the proportions shown in Table 1, below.

**Table 1**

| **Chemical** | **Formula** | **Solubility g/100 mL** | **NMC-333** | **NMC-532** | **NMC-622** | **NMC-811** |
|---|---|---|---|---|---|---|
| Li nitrate | LiNO3 | 324 | 16.34 mL | 16.34 mL | 16.34 mL | 16.34 mL |
| Ni nitrate | Ni(NO3)2 | 188 | 29.15 mL | 48.59 mL | 58.31 mL | 38.87 mL |
| Mn nitrate | Mn(NO3)2 | 206 | 26.06 mL | 28.93 mL | 17.37 mL | 2.89 mL |
| Co nitrate | Co(NO3)2 | 300 | 18.29 mL | 12.20 mL | 12.20 mL | 1.21 mL |
| Total precursor solution | | | 89.85 | 106.06 | 104.22 | 59.33 mL |
| Total loading of NMC in solution | | | 11.13 mol/L | 9.43 mol/L | 9.60 mol/L | 16.86 mol/L |
| Total loading of NMC in solution | | | 1009.52 g/L | 927.48 g/L | 907.30 g/L | 1490.69 g/L |

As can be seen in Table 1, the precursor solution for generating NMC-33 can include an aqueous solution of 16.34 mL, lithium nitrate, 29.15 mL nickel nitrate, 26.06 mL manganese nitrate, and 18.29 mL cobalt nitrate. A list of example precursor solution proportions of lithium nitrate, nickel nitrate, manganese nitrate, and cobalt nitrate for generating NMC-532, NMC-622, and NMC-811 is also provided above in Table 2. One example precursor solution for generating NMC--532 includes an aqueous solution of 16.34 mL of lithium nitrate, 48.59 mL of nickel nitrate, 28-93 mL of manganese nitrate, and 12.20 mL of cobalt nitrate. An example precursor solution for generating NMC-622 includes an aqueous solution of 16.34 mL of lithium nitrate, 58.31 mL of nickel nitrate, 17 37 mL of manganese nitrate, and 12.20 mL of cobalt nitrate. An example precursor solution for generating NMC-811 includes an aqueous solution of about 16.34 mL of lithium nitrate, about 38.87 mL of nickel nitrate, about 2.89 mL of manganese nitrate, and about 1.22 mL of cobalt nitrate.

One of the many advantages of the present technology is the ability to customize the stoichiometry of the lithium-containing battery materials. By changing one or more of the relative proportions, additives, or precursors, one can tailor the composition, purity, and/or phase of a material. In addition, one can easily manufacture more than one composition by simply altering the stoichiometry of the solution precursor (e.g., NMC-532 vs. NMC-622, LiMn₂O₄, LiNi_{0.8}Co_{0.15}Al_{0.05}O₂, LiMn_{1.5}Ni_{0.5}O₄, LiCoO₂, LiNiO₂ etc.) on a single commercial platform. Further, gradients or layers of compositional changes are possible with fluctuations or control over the precursor materials and/or additives. The continuous nature of the processes described in this disclosure allows for a layer-by-layer build-up of the cathode materials such that the chemical composition of each layer can be varied throughout its thickness to exploit the benefits of any desired material being a continuous process also eliminates batch-to-batch variations which occur in conventional battery production techniques. Additionally, the simplicity of the process allows for rapid material development and the exploration of the benefits of new material formulations (e.g., the wide range of NMC formulations and/or the addition of dopants) which may not he possible or cost effective with conventional production techniques.

According to some embodiments, the system described in FIG. 5 or a similar system can also be used to create core-shell structures, such as carbon coated particles. Other coatings or shells, such as alumina coatings, can also be produced using the techniques described herein. In other embodiments, different types of battery materials can be coated or layered onto a substrate, such as the current collector of a battery. These materials can be deposited in discrete layers having desired thicknesses, or as a continuously graded coating where the material composition of the coating gradually changes throughout the thickness of the coating. These different materials can be deposited by controlling the composition of the initial precursor solution, in some embodiments.

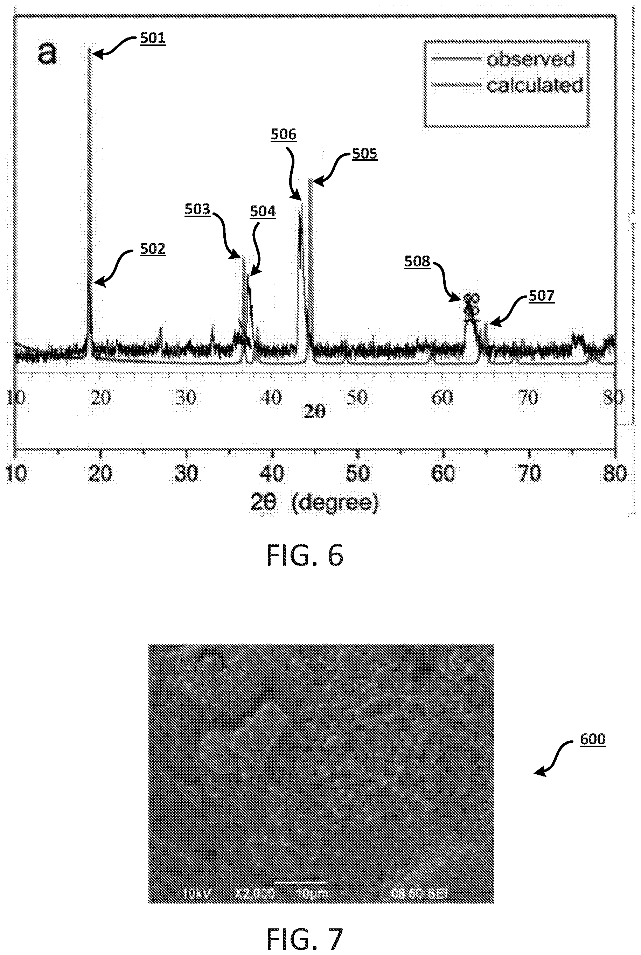

FIG. 6 is a graph of the X-ray diffraction pattern of lithium-containing particles prepared according to a non-optimized embodiment of the present disclosure. This graph corresponds to a non-optimized process for generating LiNi_{0.5}Mn_{0.3}Co_{0.2}O₂ (NMC-532) in a plasma atmosphere of O₂ using a precursor solution of lithium, nickel, manganese, and cobalt salts. In this particular example, the precursor solution included a mixture of lithium nitrate, nickel nitrate, manganese nitrate, and cobalt nitrate. The graph of FIG. 6 includes four calculated X-ray diffraction spectroscopy (XRD) peaks 501, 503, 505, and 507 corresponding to the crystalline phase of NMC 532. Even in a non-optimized process, the observed plot includes four peaks 502, 504, 506, and 508 that substantially correspond to the reference peaks 501, 503, 505, and 507.

The desired amounts of nickel and cobalt were substantially achieved, while a percentage of lithium was lost in the process and a minor amount of excess manganese was produced. A possible solution to the loss of lithium and excess manganese could be to enrich the precursor solution with about 66% extra lithium nitrate and a slightly less amount of manganese nitrate in order to tailor the process to achieve the desired NMC-532. As discussed above, the power density of the plasma, quenching rate, quenching temperature, residence time, etc. may all be controlled in order to fine tune the particles produced.

FIG. 7 shows an enlarged image 600 of the example lithium-containing particles of FIG. 6. The final lithium-containing particles and the morphology of the final particles can be tailored by controlling the residence time of the droplets within the plasma and/or optimizing the precursor solution chemistry. For example, an amorphous phase may be decreased or eliminated by increasing the residence time at a particular temperature.

In another embodiment, Table 2 shows the loading of solution to make NMC-333 using nitrates. In this particular example, the far right column of Table 2 shows the amount of solution, in mL, for lithium nitrate, nickel nitrate, manganese nitrate, and cobalt nitrate. The sum of these salt solutions for NMC-333 is 97.94 mL/mol, or 984 g/L.

**Table 2**

| **Chemical** | **Solubility** | **Molar Mass** | **Molar Solubility** | **Molar Solubility** | **mL for NMC-333** |
|---|---|---|---|---|---|
| Li nitrate | 324 g/100mL | 52.95 g/mol | 6.12 mol/100mL | 16.34 mL/mol | 16.34 |
| Ni nitrate | 188 g/100mL | 182.7 g/mol | 1.03 mol/100mL | 97.18 mL/mol | 32.36 |
| Mn nitrate | 206 g/100mL | 178.95 g/mol | 1.15 mol/100mL | 86.87 mL/mol | 28.93 |
| Co nitrate | 300 g/100mL | 182.94 g/mol | 1.64 mol/100mL | 60.98 mL/mol | 20.31 |

According to example embodiments, the techniques described herein can be used to create core-shell structures. After battery materials have exited the plasma and been quenched to a target particle size, shell materials can be coated onto them. The shell materials can be made starting from either a liquid or gas phase route, in some embodiments. An example of carbon coating through a gas phase route can begin by depositing carbon onto the surface of battery materials utilizing acetylene gas (C₂H₂) mixed with oxygen. To make the carbon, the mixture of acetylene gas and oxygen may be oxygen starved, for example using a 2: 1 acetylene to oxygen ratio. The gas mixture can be introduced into a reactor chamber containing flowing airborne battery material particles after they have exited the plasma and been quenched. The mixture of battery material particles, acetylene, and oxygen can be directed through a nozzle, and at the exit of the nozzle a stream of battery materials and gasses can flow through a flame or heat source. The heat and oxygen can decompose the acetylene to form carbon, some of which will form a shell on the surface of the battery material particles. By controlling various parameters, such as the concentration of acetylene and oxygen, battery material particles per unit volume, nozzle size, and/or nozzle exit velocity, the thickness of the shell can be tuned according to application needs without modifying the properties or morphology of the core battery material particles.

In some embodiments, carbon shells can also be formed from the acetylene/oxygen gas mixture by directing the flow of quenched battery materials through or in front of an acetylene/oxygen flame. As the cold particles pass through or in front of the flame, carbon generated within the flame can deposit onto the surface of the particles. By controlling the number of battery materials per unit volume, the velocity of the battery materials, the acetylene/oxygen ratio, the acetylene/oxygen volumetric flow rate, and the morphology of the acetylene/oxygen flame, the thickness of the shell can be tuned to target specifications.

Shell materials may also be coating onto battery material particles using a liquid precursor after the particles have exited the plasma and have been quenched to the target particle size. For example, an atomized mist of monosaccharides such as glucose or disaccharides such as sucrose are dissolved within an inorganic solvent such as water or organic solvent such as methanol. After the particles have exited the plasma and been quenched, as described above, this solution can be sprayed onto the particles using an atomizer to coat the particles with the carbon containing liquid. These coated particles can then enter an oxygen-containing hot zone between about 300-800°C. Within the hot zone, the organic or inorganic solvent can evaporate, leaving a film of saccharides on the surface of the battery materials. As the temperature of the saccharides increases above their pyrolysis temperature, carbon is left behind and a carbon shell is formed. In some embodiments, the porosity and thickness of the carbon shell can be tuned or tailored by controlling the type of saccharide and its concentration within the solvent, the type of solvent, the pressure driving the solution through the atomizer, the orientation of the atomizer(s) within the reaction chamber, oxygen concentration, and temperature within the hot zone.

Non-carbon coatings, such as alumina (Al₂O₃) can also be made using a liquid precursor in a similar manner as the carbon coatings described above. In one example embodiment, a liquid precursor is an inorganic solvent such as water or organic solvent such as methanol containing a dissolved aluminum precursor, such as aluminum nitrate in a well-defined concentration. The solution can be sprayed onto quenched particles using an atomizer in the post-plasma reactor to coat the particles with the aluminum nitrate containing liquid. These coated particles can then enter an oxygen-containing hot zone between about 200-1000°C. Within the hot zone, the solvent is evaporated leaving a film of aluminum nitrate on surface of the particles. As the temperature of the aluminum nitrate increases above its pyrolysis temperature, amorphous aluminum oxide is formed on the surface of the battery material particles. If desired, upon reaching its crystallization temperature, crystalline aluminum oxide can be formed in the range of about 400-1000°C. In some embodiments, the porosity and thickness of the alumina shell can be tuned or tailored by controlling various parameters, such as the type of aluminum salt, the concentration of the aluminum salt within the solvent, the type of solvent, the pressure driving the solution through the atomizer, the orientation of atomizer(s) within the reaction chamber, the oxygen concentration within the hot zone, and/or the temperature within the hot zone.

**In** some embodiments, the performance of a lithium-ion battery can be determined by the formulation of the cathode material. For example, spinel lithium manganese oxide (LiMn₂O₂) cathode materials allows for a high discharge rate but exhibits relatively low energy density, which can be beneficial for power tools where the batteries can be quickly changed as the usable energy is depleted. Lithium nickel cobalt aluminum oxide (NCA: LiNi_{0.8}Co_{0.015}Al_{0.05}O₂) cathode materials can be well suited for high energy applications and moderate discharge rates, like electric vehicles. Lithium cobalt oxide (LCO: LiCoO₂) cathode materials have high energy density, allowing for smaller/lighter batteries, but low discharge rates and thus may be well suited to applications like cell phones and laptop computers. Incorporating various cathode material compositions within a graded cathode battery may allow for hybrid properties with a wider range of applications. In some embodiments, layered cathodes may be produced where one material is deposited onto another with discrete interfaces. In other embodiments, a continuously variable cathode may be produced where the composition of the cathode continuously transitions from one material to the next without a discrete interface between materials.

**In** another example embodiment, multiple aqueous precursor solutions can be made from metal salts. Each precursor solution can exhibit the stoichiometry/composition of a different desired cathode material and can be loaded into its own droplet maker associated with its own plasma. For example, a first precursor solution can contain lithium acetate and manganese acetate to make spinel LMO. A second precursor solution can contain lithium acetate, nickel acetate, cobalt acetate, and aluminum nitrate to make NCA. A third precursor solution can contain lithium acetate and cobalt acetate to make LCO. In one example embodiment, a continuous coating process can be implemented to feed the spinel LMO precursor solution into a first droplet maker which is injected through a first plasma and deposited onto a current collector, which can be made of aluminum, for example. In some cases, the current collector can be moved forward and rastered back and forth under the plasma so that the entire current collector is covered. Controlling the rate and pattern of movement of the current collector and/or the precursor feed rate can determine film thickness. Once the desired thickness of spinel LMO has been deposited, the coated current collector can enter the zone of NCA deposition, where the NCA precursor solution is injected into a second plasma using a second droplet maker. Subsequently, the current collector can enter the zone of LCO deposition, where the LCO precursor solution is injected into a third plasma using a third droplet maker. After exiting the LCO coating region, the battery cathode can be ready for installation within a battery. The thickness of each layer can be independently controlled, in some embodiments, by tuning the precursor feed rate and/or controlling the rate and pattern of movement of the current collector. In one example embodiment, the desired thickness of each layer will be between about 34-100 um thick with a combined thickness of about 100-300 um.

**In** another embodiment, different coatings can be produced and deposited using a single droplet maker and plasma. For example, a current collector can be first coated in a layer of spinel LMO, and then the droplet maker can be filled with an NCA precursor solution in order to deposit a layer of NCA on top of the spinel LMO layer. Subsequently, when the NCA layer is built to a desired thickness, the droplet maker can be filled with an LCO precursor solution and the process continues until the current collector is coated with a layer of LCO. In this example embodiment, the thickness of each layer can be independently controlled by the precursor feed rate and the current collector feed rate.

**In** another example embodiment, a continuously graded cathode structure can be produced using the techniques described herein. For example, the composition of the precursor solution entering the droplet maker can be continuously varied throughout the coating process in order to gradually change the composition of the cathode coating. This can be achieved in some embodiments utilizing a set of constituent liquid precursors by continuously varying the solution precursor chemistry. In one such example, the cathode composition of the current collector can be continuously varied from LiMn_{1.5}Ni_{0.5}O₄ to LiMn₂O₄ at the interface between the cathode and electrolyte. Three separate vessels can each be filled with a single constituent metallic salt including lithium acetate, manganese acetate, or nickel acetate. A fourth mixing vessel can be fed from the three single-constituent vessels, and this fourth mixing vessel can be steadily stirred to make a homogeneous solution of the three metallic salt solutions. The mixing vessel can be first filled with stoichiometric ratios of metallic salt solutions required to form LiMn_{1.5}Ni_{0.5}O₄. This solution can be used to make the first cathode layer, which may range from about 1-50 um on a current collector with a well-defined length and width. After the first layer has been deposited, the solution volume within the fourth mixing vessel can be kept constant by continuously replenishing the mixing vessel with equal molar amounts of lithium acetate precursor and manganese acetate precursor at a rate equal to the molar flow rate exiting the droplet maker. By not replenishing the nickel salt, the concentration of nickel in the end coating can continuously decrease throughout the coating process. In some embodiments, the volume of precursor within the mixing vessel can be determined based on the volume of material required to make a cathode (or other battery component) with a defined length, width and thickness.

In additional embodiments, rather than depositing two or more different cathode materials onto a single substrate, a continuous process can be used to deposit or build cathode, anode, and solid electrolyte materials. For example, after one or more layers of cathode materials are deposited onto a substrate, as described above, the cathode can be positioned under a plasma configured to deposit solid electrolyte materials directly onto the cathode layer. Once a desired thickness of the solid electrolyte material is built up, then the cathode/electrolyte layers can be positioned under a plasma configured to deposit anode materials. Once the anode materials have been deposited, a current collector can be attached onto the anode with an binder, such as styrene butadiene copolymer or polyvinyldene fluoride.

Exemplary flowcharts are provided herein for illustrative purposes and are non-limiting examples of methods. One of ordinary skill in the art will recognize that exemplary methods may include more or fewer steps than those illustrated in the exemplary flowcharts, and that the steps in the exemplary flowcharts may be performed in a different order than the order shown in the illustrative flowcharts.

In describing exemplary embodiments, specific terminology is used for the sake of clarity. For purposes of description, each specific term is intended to at least include all technical and functional equivalents that operate in a similar manner to accomplish a similar purpose. Additionally, in some instances where a particular exemplary embodiment includes a plurality of system elements, device components or method steps, those elements, components or steps may be replaced with a single element, component or step. Likewise, a single element, component or step may be replaced with a plurality of elements, components or steps that serve the same purpose. Moreover, while exemplary embodiments have been shown and described with references to particular embodiments, those of ordinary skill in the art will understand that various substitutions and alterations in form and detail may be made therein without departing from the scope of the invention.

## Claims

1. A method for generating lithium ion battery materials comprising:
combining starting materials to form a homogeneous precursor solution including lithium;
generating droplets with controlled size of the homogeneous precursor solution using a droplet maker;
introducing the droplets of the homogeneous precursor solution into a microwave generated plasma;
producing micron or sub-micron scale lithium-containing particles from the microwave generated plasma;
collecting the lithium-containing particles; and
forming a slurry with the lithium-containing particles to form lithium ion battery materials.

2. The method of claim 1, wherein collecting the lithium-containing particles includes quenching the lithium-containing particles, the method further comprising:
controlling a quenching rate of the lithium-containing particles by selecting a quenching fluid, controlling a quenching fluid flow speed, or controlling a quenching fluid temperature.

3. The method of claim 1, further comprising:
controlling a size of the droplets of the homogeneous precursor solution using the droplet maker; or
controlling a residence time of the droplets of the homogeneous precursor solution within the microwave generated plasma by controlling at least one of: a plasma gas flow velocity, a power density of the microwave generated plasma, or a velocity of the droplets exiting the droplet maker.

4. The method of claim 1, wherein the homogeneous precursor solution includes an aqueous solution of hydrated or non-hydrated forms of lithium acetate, nickel acetate, manganese acetate, and cobalt acetate; or includes an aqueous solution of lithium nitrate, nickel nitrate, manganese nitrate, and cobalt nitrate.

5. The method of claim 1, wherein generating droplets with controlled size includes generating two or more streams of droplets having different diameters.

6. The method of claim 1, where the microwave generated plasma is generated in oxygen gas or an oxygen-containing gas.

7. The method of claim 1 for tailoring lithium ion battery materials, wherein collecting the lithium-containing particles involves
quenching the lithium-containing particles; the method further comprising:
tailoring at least one of: porosity, morphology, particle size, particle size distribution, or chemical composition of the lithium-containing particles by controlling at least one of: precursor solution chemistry, droplet size, plasma gas flow rates, residence time of the droplets within the microwave generated plasma, quenching rate, or power density of the microwave generated plasma.

8. The method of claim 7, wherein tailoring the morphology of the lithium-containing particles includes controlling the residence time of the droplets within the microwave generated plasma and an afterglow region of the microwave generated plasma; or wherein controlling the porosity of the lithium ion particles includes controlling at least one of: amounts of nitrate materials and acetate materials within the homogeneous precursor solution, solution precursor chemistry, or the residence time of the droplets within the microwave generated plasma; or wherein controlling the chemical composition of the lithium-containing particles includes controlling proportions of the starting materials within the precursor solution; or wherein controlling the particle size of the lithium ion particles includes at least one of: controlling the droplet size of the droplets of the precursor solution, or controlling a concentration of starting materials within the precursor solution.

9. The method of claim 7, wherein generating droplets with controlled size includes generating two or more streams of droplets having different diameters.

10. The method of claim 9, wherein the two or more streams of droplets are generated using different nozzles or openings in the droplet maker.

11. The method of claim 7, wherein tailoring the chemical composition of the lithium ion particles includes:
determining a desired chemical composition of the lithium ion particles prior to forming the homogeneous precursor solution; and
calculating stoichiometric proportions of the starting materials based on the desired chemical composition of the lithium ion particles.

12. The method of claim 1, wherein the lithium-containing particles have the formula:
LiNiₓMn_{y}Co_{z}O₂
wherein x ≥ 0, y ≥ 0, z ≥ 0, and x+y+z=1;
and wherein combining the starting materials comprises dissolving a combination of lithium salt, nickel salt, manganese salt, and cobalt salt in a solvent to form a homogeneous precursor solution.

13. The method of claim 12, wherein generating droplets with controlled size includes generating two or more streams of droplets having different diameters in order to generate a multi-modal particle size distribution among the particles of LiNiₓMn_{y}Co_{z}O₂.

14. The method of claim 1, wherein the lithium-containing particles have the formula:
LiNiₓCo_{y}Al_{z}O₂
wherein x = ~ 0.8, y = ~ 0.15, z = ~ 0.05;
and wherein combining the starting materials comprises dissolving a combination of lithium salt, nickel salt, cobalt salt, and aluminum salt in a solvent to form a homogeneous precursor solution.

15. The method of claim 14, wherein generating droplets with controlled size includes generating two or more streams of droplets having different diameters in order to generate a multi-modal particle size distribution among the particles of LiNiₓCo_{y}Al_{z}O₂.

## Patentansprüche

1. Verfahren zum Generieren von Lithium-Ionen-Batteriematerialien, das Folgendes umfasst:
Kombinieren von Ausgangsmaterialien, um eine homogene Vorläuferlösung, die Lithium beinhaltet, auszubilden;
Generieren von Tröpfchen mit gesteuerter Größe aus der homogenen Vorläuferlösung unter Verwendung eines Tröpfchenerzeugers;
Einbringen der Tröpfchen aus der homogenen Vorläuferlösung in ein durch Mikrowellen generiertes Plasma;
Produzieren von lithiumhaltigen Partikeln in einem Mikrometer- oder Submikrometermaßstab aus dem durch Mikrowellen generierten Plasma;
Sammeln der lithiumhaltigen Partikel; und
Ausbilden einer Aufschlämmung mit den lithiumhaltigen Partikeln, um Lithium-Ionen-Batteriematerialien auszubilden.

2. Verfahren nach Anspruch 1, wobei das Sammeln der lithiumhaltigen Partikel ein Quenchen der lithiumhaltigen Partikel beinhaltet, wobei das Verfahren ferner Folgendes umfasst:
Steuern einer Quenchrate der lithiumhaltigen Partikel durch Auswählen eines Quenchfluids, Steuern einer Quenchfluidfließschnelligkeit oder Steuern einer Quenchfluidtemperatur.

3. Verfahren nach Anspruch 1, das ferner Folgendes umfasst:
Steuern einer Größe der Tröpfchen aus der homogenen Vorläuferlösung unter Verwendung des Tröpfchenerzeugers; oder
Steuern einer Verweilzeit der Tröpfchen aus der homogenen Vorläuferlösung innerhalb des durch Mikrowellen generierten Plasmas durch Steuern von Folgendem: einer Plasmagasfließgeschwindigkeit, einer Leistungsdichte des durch Mikrowellen generierten Plasmas und/oder einer Geschwindigkeit der Tröpfchen, die den Tröpfchenerzeuger verlassen.

4. Verfahren nach Anspruch 1, wobei die homogene Vorläuferlösung eine wässrige Lösung von hydratisierten oder nicht-hydratisierten Formen von Lithiumazetat, Nickelazetat, Manganazetat und Kobaltazetat beinhaltet; oder eine wässrige Lösung von Lithiumnitrat, Nickelnitrat, Mangannitrat und Kobaltnitrat beinhaltet.

5. Verfahren nach Anspruch 1, wobei das Generieren von Tröpfchen mit gesteuerter Größe das Generieren von zwei oder mehr Strömen von Tröpfchen, die unterschiedliche Durchmesser aufweisen, beinhaltet.

6. Verfahren nach Anspruch 1, wobei das durch Mikrowellen generierte Plasma in Sauerstoffgas oder einem sauerstoffhaltigen Gas generiert wird.

7. Verfahren nach Anspruch 1 zum Maßschneidern von Lithium-Ionen-Batteriematerialien, wobei das Sammeln der lithiumhaltigen Partikel Folgendes involviert:
Quenchen der lithiumhaltigen Partikel; wobei das Verfahren ferner Folgendes umfasst:
Maßschneidern von Folgendem: Porosität, Morphologie, Partikelgröße, Partikelgrößenverteilung und/oder chemischer Zusammensetzung der lithiumhaltigen Partikel durch Steuern von Folgendem: Vorläuferlösungschemie, Tröpfchengröße, Plasmagasfließraten, Verweilzeit der Tröpfchen innerhalb des durch Mikrowellen generierten Plasmas, Quenchrate und/oder Leistungsdichte des durch Mikrowellen generierten Plasmas.

8. Verfahren nach Anspruch 7, wobei das Maßschneidern der Morphologie der lithiumhaltigen Partikel das Steuern der Verweilzeit der Tröpfchen innerhalb des durch Mikrowellen generierten Plasmas und einer Nachleuchtregion des durch Mikrowellen generierten Plasmas beinhaltet; oder wobei das Steuern der Porosität der Lithium-Ionen-Partikel das Steuern von Folgendem beinhaltet: Mengen von Nitratmaterialien und Azetatmaterialien innerhalb der homogenen Vorläuferlösung, Vorläuferlösungschemie und/oder der Verweilzeit der Tröpfchen innerhalb des durch Mikrowellen generierten Plasmas; oder wobei das Steuern der chemischen Zusammensetzung der lithiumhaltigen Partikel das Steuern von Anteilen der Ausgangsmaterialien innerhalb der Vorläuferlösung beinhaltet; oder wobei das Steuern der Partikelgröße der Lithium-Ionen-Partikel Folgendes beinhaltet: Steuern der Tröpfchengröße der Tröpfchen aus der Vorläuferlösung und/oder Steuern einer Konzentration von Ausgangsmaterialien innerhalb der Vorläuferlösung.

9. Verfahren nach Anspruch 7, wobei das Generieren von Tröpfchen mit gesteuerter Größe das Generieren von zwei oder mehr Strömen von Tröpfchen, die unterschiedliche Durchmesser aufweisen, beinhaltet.

10. Verfahren nach Anspruch 9, wobei die zwei oder mehr Ströme von Tröpfchen unter Verwendung von unterschiedlichen Düsen oder Öffnungen in dem Tröpfchenerzeuger generiert werden.

11. Verfahren nach Anspruch 7, wobei das Maßschneidern der chemischen Zusammensetzung der Lithium-Ionen-Partikel Folgendes beinhaltet:
Bestimmen einer gewünschten chemischen Zusammensetzung der Lithium-Ionen-Partikel vor dem Ausbilden der homogenen Vorläuferlösung; und
Berechnen stöchiometrischer Anteile der Ausgangsmaterialien basierend auf der gewünschten chemischen Zusammensetzung der Lithium-Ionen-Partikel.

12. Verfahren nach Anspruch 1, wobei die lithiumhaltigen Partikel die folgende Formel aufweisen:
LiNiₓMn_{y}Co_{z}O₂
wobei x ≥ 0, y ≥ 0, z ≥ 0 und x+y+z=1;
und wobei das Kombinieren der Ausgangsmaterialien ein Auflösen einer Kombination aus Lithiumsalz, Nickelsalz, Mangansalz und Kobaltsalz in einem Lösungsmittel, um eine homogene Vorläuferlösung auszubilden, umfasst.

13. Verfahren nach Anspruch 12, wobei das Generieren von Tröpfchen mit gesteuerter Größe das Generieren von zwei oder mehr Strömen von Tröpfchen, die unterschiedliche Durchmesser aufweisen, um eine multimodale Partikelgrößenverteilung zwischen den Partikeln von LiNiₓMn_{y}Co_{z}O₂ zu generieren, beinhaltet.

14. Verfahren nach Anspruch 1, wobei die lithiumhaltigen Partikel die folgende Formel aufweisen:
LiNiₓCo_{y}Al_{z}O₂
wobei x = ∼0,8, y = -0,15, z = -0,05;
und wobei das Kombinieren der Ausgangsmaterialien das Auflösen einer Kombination aus Lithiumsalz, Nickelsalz, Kobaltsalz und Aluminiumsalz in einem Lösungsmittel, um eine homogene Vorläuferlösung auszubilden, umfasst.

15. Verfahren nach Anspruch 14, wobei das Generieren von Tröpfchen mit gesteuerter Größe das Generieren von zwei oder mehr Strömen von Tröpfchen, die unterschiedliche Durchmesser aufweisen, um eine multimodale Partikelgrößenverteilung zwischen den Partikeln von LiNiₓCo_{y}Al_{z}O₂ zu generieren, beinhaltet.

## Revendications

1. Procédé permettant de générer des matériaux de batterie au lithium-ion comprenant :
le fait de combiner des matériaux de départ pour former une solution de précurseur homogène comprenant du lithium ;
la génération de gouttelettes avec une taille contrôlée de la solution de précurseur homogène à l'aide d'un générateur de gouttelettes ;
l'introduction des gouttelettes de la solution de précurseur homogène dans un plasma généré par micro-ondes ;
la production de particules contenant du lithium de l'ordre du micron ou du sousmicron à partir du plasma généré par micro-ondes ;
la collecte des particules contenant du lithium ; et
la formation d'une bouillie avec les particules contenant du lithium pour former des matériaux de batterie au lithium-ion.

2. Procédé selon la revendication 1, dans lequel la collecte des particules contenant du lithium comporte la trempe des particules contenant du lithium, le procédé comprenant en outre :
le contrôle d'une vitesse de trempe des particules contenant du lithium en sélectionnant un fluide de trempe, le contrôle d'une vitesse d'écoulement du fluide de trempe ou le contrôle d'une température de fluide de trempe.

3. Procédé selon la revendication 1, comprenant en outre :
le contrôle d'une taille des gouttelettes de la solution de précurseur homogène à l'aide du générateur de gouttelettes ; ou
le contrôle d'un temps de séjour des gouttelettes de la solution de précurseur homogène dans le plasma généré par micro-ondes en contrôlant : une vitesse d'écoulement du gaz plasma et/ou une densité de puissance du plasma généré par micro-ondes et/ou une vitesse des gouttelettes sortant du générateur de gouttelettes.

4. Procédé selon la revendication 1, dans lequel la solution de précurseur homogène comporte une solution aqueuse de formes hydratées ou non hydratées d'acétate de lithium, d'acétate de nickel, d'acétate de manganèse et d'acétate de cobalt ; ou comporte une solution aqueuse de nitrate de lithium, de nitrate de nickel, de nitrate de manganèse et de nitrate de cobalt.

5. Procédé selon la revendication 1, dans lequel la génération de gouttelettes avec une taille contrôlée comporte la génération de deux ou plusieurs flux de gouttelettes ayant des diamètres différents.

6. Procédé selon la revendication 1, dans lequel le plasma généré par micro-ondes est généré dans du gaz oxygène ou dans un gaz contenant de l'oxygène.

7. Procédé selon la revendication 1 pour adapter des matériaux de batterie au lithium-ion, dans lequel la collecte des particules contenant du lithium implique
la trempe des particules contenant du lithium ; le procédé comprenant en outre :
le fait d'adapter : la porosité et/ou la morphologie et/ou la taille des particules et/ou la distribution de la taille des particules et/ou la composition chimique des particules contenant du lithium en contrôlant : la chimie de la solution de précurseur et/ou la taille des gouttelettes et/ les débits de gaz plasma et/ou le temps de séjour des gouttelettes dans le plasma généré par micro-ondes et/ou la vitesse de trempe et/ou la densité de puissance du plasma généré par micro-ondes.

8. Procédé selon la revendication 7, dans lequel le fait d'adapter la morphologie des particules contenant du lithium comporte le contrôle du temps de séjour des gouttelettes dans le plasma généré par micro-ondes et dans une région de postluminescence du plasma généré par micro-ondes ; ou dans lequel le contrôle de la porosité des particules d'ion lithium comporte le contrôle : des quantités de matériaux nitrate et de matériaux acétate dans la solution de précurseur homogène et/ou la chimie du précurseur de solution et/ou le temps de séjour des gouttelettes dans le plasma généré par micro-ondes ; ou dans lequel le contrôle de la composition chimique des particules contenant du lithium comporte le contrôle des proportions des matériaux de départ dans la solution de précurseur ; ou dans lequel le contrôle de la taille des particules d'ion lithium comporte : le contrôle de la taille des gouttelettes des gouttelettes de la solution de précurseur et/ou le contrôle d'une concentration de matières de départ dans la solution de précurseur.

9. Procédé selon la revendication 7, dans lequel la génération de gouttelettes avec une taille contrôlée comporte la génération de deux ou plusieurs flux de gouttelettes ayant des diamètres différents.

10. Procédé selon la revendication 9, dans lequel les deux ou plusieurs flux de gouttelettes sont générés à l'aide des différentes buses ou ouvertures dans le générateur de gouttelettes.

11. Procédé selon la revendication 7, dans lequel le fait d'adapter la composition chimique des particules d'ion lithium comporte :
la détermination d'une composition chimique souhaitée des particules d'ion lithium avant de former la solution de précurseur homogène ; et
le calcul des proportions stœchiométriques des matières de départ sur la base de la composition chimique souhaitée des particules d'ion lithium.

12. Procédé selon la revendication 1, dans lequel les particules contenant du lithium ont la formule :
LiNiₓMn_{y}Co_{z}O₂
où x ≥ 0, y ≥ 0, z ≥ 0, et x + y + z = 1 ;
et la combinaison des matériaux de départ comprenant la dissolution d'une combinaison de sel de lithium, de sel de nickel, de sel de manganèse et de sel de cobalt dans un solvant pour former une solution de précurseur homogène.

13. Procédé selon la revendication 12, dans lequel la génération de gouttelettes avec une taille contrôlée comporte la génération de deux ou plusieurs flux de gouttelettes ayant des diamètres différents afin de générer une distribution multimodale de la taille des particules parmi les particules de LiNiₓMn_{y}Co_{z}O₂.

14. Procédé selon la revendication 1, dans lequel les particules contenant du lithium ont la formule :
LiNiₓCo_{y}Al_{z}O₂
où x = ~ 0,8, y = ~ 0,15, z = ~ 0,05 ;
et la combinaison des matériaux de départ comprenant la dissolution d'une combinaison de sel de lithium, de sel de nickel, de sel de cobalt et de sel d'aluminium dans un solvant pour former une solution de précurseur homogène.

15. Procédé selon la revendication 14, dans lequel la génération de gouttelettes avec une taille contrôlée comporte la génération de deux ou plusieurs flux de gouttelettes ayant des diamètres différents afin de générer une distribution multimodale de la taille des particules parmi les particules de LiNiₓCo_{y}Al_{z}O₂.
